(19) Europäisches Patentamt

European Patent Office

Office européen des brevets

(11) **EP 0 826 380 B1**

(12) **EUROPÄISCHE PATENTSCHRIFT**

(45) Veröffentlichungstag und Bekanntmachung des Hinweises auf die Patenterteilung:
**30.10.2002 Patentblatt 2002/44**

(51) Int Cl.⁷: **A61L 15/58**, A61F 13/02, C09J 7/02, C09J 7/04

(21) Anmeldenummer: **97112605.7**

(22) Anmeldetag: **23.07.1997**

(54) **Selbstklebend ausgerüstete Trägermaterialien**

Self-adhesive support materials

Matériaux de support auto-adhésifs

(84) Benannte Vertragsstaaten:
**AT DE ES FR GB IT NL SE**

(30) Priorität: **06.08.1996 DE 19631422**

(43) Veröffentlichungstag der Anmeldung:
**04.03.1998 Patentblatt 1998/10**

(73) Patentinhaber: **Beiersdorf Aktiengesellschaft**
**20245 Hamburg (DE)**

(72) Erfinder:
 • **Himmelsbach, Peter**
 **21614 Buxtehude (DE)**
 • **Jauchen, Peter**
 **22455 Hamburg (DE)**
 • **Albrod, Andreas, Dr.**
 **21217 Seevetal (DE)**
 • **Andrews, Arthur-Hugh**
 **25337 Kölln-Reisiek (DE)**
 • **Keite-Telgenbüscher, Klaus, Dr.**
 **22335 Hamburg (DE)**
 • **Leutz, Reiner**
 **21465 Reinbek (DE)**
 • **Scharnhorst, Christian**
 **25491 Hetlingen (DE)**
 • **Stradt, Thorsten**
 **22763 Hamburg (DE)**
 • **Kummer, Andreas B., Dr.**
 **21079 Hamburg (DE)**
 • **Trotter, Sebastian**
 **21244 Buchholz (DE)**

(56) Entgegenhaltungen:
EP-A- 0 443 263         US-A- 5 453 319

**Beschreibung**

Die Erfindung betrifft mindestens einseitig selbstklebend ausgerüstete Trägermaterialien, die mit einer Haftschmelzklebemasse ganz oder teilweise beschichtet worden sind, sowie deren Verwendung.

Stark klebende orthopädische Bandagen und andere medizinische Produkte werden üblicherweise vollflächig mit einer Zink-Kautschuk-Klebemasse beschichtet. Das Verkleben derartiger Produkte auf der Haut zeigt nach dem Ablösen deutliche Hautirritationen und eine mechanische Beanspruchung der Haut. Die Verklebung läßt sich ohne Hilfsmittel nur unter Schmerzen lösen.
Fallweise kommt es zu allergischen Reaktionen. Die verwendeten Klebemassen führen darüber hinaus oft zu einem Massetransfer, d.h. einem Umspulen, auf die Haut.

Die Verwendung von hautfreundlichen Klebemassen, wie Acrylatklebemassen, ist aufgrund der geringen Scherstabilität und Anfaßklebrigkeit nicht erwägenswert. Eine Verbesserung durch eine Nachbehandlung, insbesondere Vernetzung, ist möglich, jedoch bleibt das Ergebnis insgesamt unbefriedigend. Weiterhin reicht die Klebkraft auf der Trägerrückseite solcher Systeme bei zirkulär angelegten Verbänden mit mehreren Lagen nicht für einen stabilen funktionalen Verband aus. Die propriorezeptive Wirkung ist gegenüber den Systemen mit einer Zink-Kautschuk-Klebemasse geringer.

Andere bekannte Klebesysteme auf Basis von herkömmlichen Blockcopolymeren sind zum einen durch hohen Stabilisatorzusatz nicht hautfreundlich bzw. zeigen durch die hohe Kohäsivität bislang nur eine Eignung für technische Anwendungsfälle, zum anderen sind sie nicht stark hautklebend und hauthaftend einzustellen.

Bei der partiellen Beschichtung zeigt sich aufgrund eines begrenzt möglichen Masseauftrags eine zu geringe Klebkraft, insbesondere bei schweren Trägermaterialien.

Die zuvor genannten Klebemassen sind druckempfindliche Selbstklebemassen, wobei die Massen für die Verarbeitung in einer Trägermatrix vorliegen können. Als Trägermatrix werden gängige organische oder anorganische Lösemittel oder Dispergiermittel verstanden.

Systeme ohne Trägermatrix werden als 100 %-Systeme bezeichnet und sind ebenfalls nicht unbekannt. Sie werden im elastischen oder thermoplastischen Zustand verarbeitet. Eine gängige Verarbeitungsweise ist die Schmelze. Auch solche Haftschmelzklebemassen sind im Stande der Technik bereits vorbeschrieben. Sie basieren auf natürlichen oder synthetischen Kautschuken und/oder anderen synthetischen Polymeren.
Aufgrund ihrer hohen Härte ist für solche 100 %-Systeme die Hauthaftung problematisch.

Vorteilhaft an den 100 %-Systemen ist, daß bei ihnen verfahrenstechnisch ein Entfernen der Trägermatrix, d.h. der Hilfsmittel vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials.

Es ist ferner bekannt, derartige Selbstklebemassen nicht nur vollflächig sondern auch rasterpunktförmig aufzubringen, beispielsweise durch Siebdruck (DE-PS 42 37 252), wobei die Klebstoffpünktchen auch unterschiedlich groß und/oder unterschiedlich verteilt sein können (EP-PS 353 972), oder durch Tiefdruck von in Längs- und Querrichtung zusammenhängenden Stegen (DE-PS 43 08 649).

Der Vorteil des rasterförmigen Auftrags zeigt sich darin, daß die Klebematerialien bei entsprechend porösem Trägermaterial luft- und wasserdampfdurchlässig sowie in der Regel leicht wieder ablösbar sind.

Ein Nachteil dieser Produkte besteht jedoch in der Tatsache, daß bei zu hoher Flächendeckung der an sich undurchlässigen Klebeschicht die Luft- und Wasserdampfdurchlässigkeit sich entsprechend verringert, sowie der Klebemassenverbrauch steigt und bei geringer Flächendeckung der Klebeschicht die Klebeeigenschaften leiden, d.h. das Produkt löst sich, insbesondere bei schweren, textilen Trägermaterialien, zu leicht vom Untergrund.

Aufgabe der Erfindung war es deshalb, die aus dem Stand der Technik bekannten Nachteile zu vermeiden und weiterhin ein mindestens einseitig selbstklebendes Trägermaterial zur Verfügung zu stellen, das aufgrund seiner Ausrüstung, also der Eigenschaften der Klebemasse, der Auftragsform sowie der Materialeigenschaften des Trägermaterials, zur funktionsgerechten Verwendung für diverse Fixierungen dient, insbesondere für medizinische Produkte.

Gelöst wird diese Aufgabe durch ein mindestens einseitig selbstklebend ausgerüstetes Trägermaterial, wobei die Selbstklebemasse eine Haftschmelzklebemasse ist, die bei einer Frequenz von 0,1 rad/s eine Glasübergangstemperatur von weniger als -3 °C, bevorzugt von -6 °C bis -30 °C, ganz besonders bevorzugt -9 °C bis -25 °C, aufweist.

Bevorzugt basiert die Klebemasse auf Blockcopolymerbasis ist, insbesondere A-B- oder A-B-A-Blockcopolymere oder deren Mischungen. Die harte Phase A ist vornehmlich Polystyrol oder dessen Derivate und die weiche Phase B Ethylen, Propylen, Butylen, Butadien, Isopren oder deren Mischungen, hierbei besonders bevorzugt Ethylen und Butylen oder deren Mischungen.
Polystyrolblöcke können aber auch in der weichen Phase B enthalten sein, und zwar bis zu 20 Gew.-%. Der gesamte Styrolanteil im Polymer liegt aber stets niedriger als 35 Gew.-%. Bevorzugt werden Styrolanteile zwischen 5 Gew.-% und 30 Gew.-%, da ein niedrigerer Styrolanteil die Klebemasse anschmiegsamer macht.
Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

In einer vorteilhaften Ausführung weist die Haftschmelzklebemasse die nachfolgend angegebene Zusammensetzung auf:

- 10 Gew.-% bis 90 Gew.-% Blockcopolymere,
- 5 Gew.-% bis 80 Gew.-% Klebrigmacher wie Öle, Wachse, Harze und/oder deren Mischungen, bevorzugt Mischungen aus Harzen und Ölen,
- weniger als 60 % Weichmacher
- weniger als 15 Gew.-% Additive
- weniger als 5 Gew.-% Stabilisatoren.

Die als Klebrigmacher dienenden Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden langkettige Fettsäuren und/ oder deren Ester Verwendung. Diese Zusätze dienen dabei der Einstellung der Klebeeigenschaften und der Stabilität.

Ein Füllen der Klebemasse mit mineralischen Füllstoffen ist möglich.

Die Haftschmelzklebemasse weist einen Erweichungspunkt oberhalb von 70 °C, bevorzugt 95 °C bis 120 °C, auf.

Insbesondere an medizinische Produkte, beispielsweise eine orthopädische Binde, werden hohe Anforderungen bezüglich der Klebeeigenschaften gestellt. Für eine ideale Anwendung sollte die Selbstklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen der Lagen kommt, eine hohe Scherfestigkeit der Selbstklebemasse notwendig.

Durch die erfindungswesentliche, gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut und der Trägerrückseite erreicht. Die hohe Scherfestigkeit der hier eingesetzten Selbstklebemasse wird durch die hohe Kohäsivität des Blockcopolymeren erreicht. Die gute Anfaßklebrigkeit ergibt sich durch die eingesetzte Palette an Klebrigmachern und Weichmachern.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur der Schmelzhaftkleber zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient (Q = tan δ) zwischen dem Verlustmodul (G'' viskoser Anteil) und dem Speichermodul (G' elastischer Anteil) ermittelt. Für das subjektive Empfinden der Anfaßklebrigkeit (Tack) wird eine hohe Frequenz gewählt, sowie für die Scherfestigkeit eine niedrige Frequenz.

Eine hoher Zahlenwert bedeutet eine bessere Anfaßklebrigkeit und eine schlechtere Scherstabilität.

Der komplexe-dynamische Glasübergangspunkt ist der Übergangspunkt vom amorphen in den viskoelastischen Bereich. Er entspricht dem Maximum der Temperaturfunktion bei vorgegebener Frequenz.

$$Q = \tan \delta = G''/G'$$

| Bezeichnung | $T_G$ niedrige Frequenz | Scherstabilität niedrige Frequenz / RT | Anfaßklebrigkeit hohe Frequenz / RT |
|---|---|---|---|
| Schmelzhaftkleber A | -12 ± 2 °C | tan δ = 0,08 ± 0,03 | tan δ = 0,84 ± 0,03 |
| Schmelzhaftkleber B | -9 ± 2 °C | tan δ = 0,22 ± 0,03 | tan δ = 1,00 ± 0,03 |

Bevorzugt werden erfindungsgemäß Haftschmelzklebemassen, bei denen das Verhältnis des viskosen Anteils zum elastischen Anteil bei einer Frequenz von 100 rad/s bei 25 °C größer 0,7 ist, oder Haftschmelzklebemassen, bei denen das Verhältnis des viskosen Anteils zum elastischen Anteil bei einer Frequenz von 0,1 rad /s bei 25 °C kleiner 0,4 ist, bevorzugt zwischen 0,35 und 0,02, ganz besonders bevorzugt zwischen 0,3 und 0,1.

Vorteilhaft insbesondere für die Verwendung bei medizinischen Produkten ist weiterhin, wenn die Haftschmelzklebemasse partiell auf dem Trägermaterial aufgetragen ist, beispielsweise durch Rasterdruck, Thermosiebdruck oder Tiefdruck, denn im Vollstrich selbstklebend ausgerüstete Trägermaterialien rufen bei der Applikation mechanische Hautirritationen hervor.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien. Hierdurch werden Hautirritationen, die durch Stauungen der Körperflüssigkeiten hervorgerufen werden, vermieden. Die angelegten Abführungskanäle ermöglichen ein Ableiten auch unter Verwendung eines mehrlagigen Verbandes.

Bevorzugt wird der Auftrag in Form von polygeometrischen Kalotten und ganz besonders von solchen Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.

Femer kann sie beispielsweise auch aufgesprüht sein, was ein mehr oder weniger unregelmäßiges Auftragsbild ergibt.

Die Selbstklebemasse kann gleichmäßig auf dem Trägermaterial verteilt sein, sie kann aber auch funktionsgerecht für das Produkt über die Fläche unterschiedlich stark oder dicht aufgetragen sein.

Das Prinzip des Thermosiebdrucks besteht in der Verwendung einer rotierenden beheizten, nahtlosen, trommelförmigen perforierten Rundschablone, die über eine Düse mit der Haftschmelzklebemasse beschickt wird. Eine speziell geformte Düsenlippe (Rundoder Vierkantrakel) preßt die über einen Kanal eingespeiste Selbstklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoffkalotten geschieht dabei nach folgendem Mechanismus:

Der Düsenrakeldruck fördert die Selbstklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Selbstklebemasse und die innere Kohäsion des Hotmelts wird von der auf dem Träger bereits haftenden Basis der Kalotten der in den Löchern begrenzte Vorrat an Haftschmelzklebemasse konturenscharf abgezogen bzw. durch den Rakeldruck auf den Träger gefördert.

Nach Beendigung dieses Transportes formt sich, abhängig von der Rheologie der Haftschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Bei der Siebschablone im Thermosiebdruck kann das Steg/Loch-Verhältnis kleiner 2:1 sein, bevorzugt kleiner oder gleich 1:1.

Der beschriebene Bildungsmechanismus der Kalotten erfordert bevorzugt saugfähige oder zumindest von Haftschmelzklebemasse benetzbare Trägermaterialien. Nichtbenetzende Trägeroberflächen müssen durch chemische oder physikalische Verfahren vorbehandelt werden. Dies kann durch zusätzliche Maßnahmen wie z.B. Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Träger, der mit größerem Durchmesser und größerer Kalottenhöhe für rauhe oder stark porige Trägermaterialien vorgesehen ist.

Die Positionierung der Kalotten auf dem Träger wird durch die in weiten Grenzen variierbare Geometrie des Auftragswerkes, z.B. Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 100 m/min, beschichtet, wobei die Beschichtungstemperatur größer als die Erweichungstemperatur zu wählen ist.

Die Haftschmelzklebemasse kann mit einem Flächengewicht von größer 15 g/m$^2$, bevorzugt zwischen 70 g/m$^2$ und 300 g/m$^2$, ganz besonders bevorzugt zwischen 90 g/m$^2$ und 160 g/m$^2$, auf dem Trägermaterial aufgetragen sein. Der prozentuale Anteil, der mit der Haftschmelzklebemasse beschichteten Fläche sollte mindestens 20 % betragen und kann bis zu ca. 95% reichen, für spezielle Produkte bevorzugt 40 % bis 60% sowie 70 % bis 95%. Dieses kann gegebenenfalls durch Mehrfachapplikation erreicht werden, wobei gegebenenfalls auch Klebemassen mit unterschiedlichen Eigenschaften eingesetzt werden können.

Das selbstklebend ausgerüstete Trägermaterial weist eine Klebkraft auf der Trägerrückseite von mindestens 1,5 N/cm auf, besonders eine Klebkraft zwischen 2,5 N/cm und 5 N/cm. Auf anderen Untergründen können höhere Klebkräfte erreicht werden.

Die Kombination der Selbstklebemasse und der partiellen Beschichtung sichert auf der einen Seite eine sichere Verklebung insbesondere des medizinischen Produktes auf der Haut, auf der anderen Seite sind zumindest visuell erkennbare allergische oder mechanische Hautirritationen ausgeschlossen, auch bei einer Anwendung, die sich über mehrere Tage erstreckt.

Die Epilation entsprechender Körperregionen und der Massetransfer auf die Haut sind aufgrund der hohen Kohäsivität des Klebers vernachlässigbar, weil der Kleber nicht an Haut und Haar verankert, vielmehr ist die Verankerung der Klebemasse auf dem Trägermaterial mit bis zu 12 N/cm (Probenbreite) sehr gut, insbesondere für medizinische Anwendungen.

Durch die ausgeformten Sollbruchstellen in der Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Weiter unterstützt die individuelle biomechanische Klebkraftsteuerung, welche eine nachweisliche Absenkung der Klebkraft dieser selbstklebend ausgerüsteten Trägermaterialien aufweist, die Ablösbarkeit. Der angelegte Verband zeigt gute propriorezeptive Wirkungen.

Je nach Trägermaterial und dessen Temperaturempfindlichkeit kann die Selbstklebemasse direkt aufgetragen sein oder zuerst auf einen Hilfsträger aufgebracht und dann auf den endgültigen Träger transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Als Trägermaterialien eignen sich alle starren und elastischen Flächengebilde aus synthetischen und natürlichen Rohstoffen. Bevorzugt sind Trägermaterialien die nach Applikation der Klebemasse so eingesetzt werden können, daß sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt.

Weiter können diese Materialien vor- bzw. nachbehandelt werden. Gängige Vorbehandlungen sind Corona und Hydrophobieren; geläufige Nachbehandlungen sind Kalandern, Tempern, Kaschieren, Stanzen und Eindecken.

Das beschichtete Trägermaterial kann eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), aufweisen, des weiteren eine Wasserdampfdurchlässigkeit von größer 500 g/(m$^2$*24h), bevorzugt größer 1000 g/(m$^2$*24h), ganz besonders bevorzugt größer 2000 g/(m$^2$*24h).

Das beschichtete Trägermaterial kann darüber hinaus auch im Lagenverbund noch eine Luftdurchlässigkeit von 1 cm$^3$/(cm$^2$*s) und eine Wasserdampfdurchlässigkeit von 500 g/(m$^2$*24h) aufweisen.

Schließlich kann das selbstklebend ausgerüstete Trägermaterial nach der Applikation eingedeckt oder mit einer Wundauflage, Polsterung versehen werden.

Besonders vorteilhaft ist, daß das selbstklebend ausgerüstete Trägermaterial sterilisierbar, bevorzugt γ-(gamma) sterilisierbar, ist. So sind besonders geeignet für eine nachträgliche Sterilisation Haftschmelzklebemassen auf Blockcopolymerbasis, welche keine Doppelbindungen enthalten. Dieses gilt ins besondere für Styrol-Butylen-Ethylen-Styrol-Blockcopolymerisate oder Styrol-Butylen-Styrol-Blockcopolymerisate. Es treten hierbei keine für die Anwendung signifikanten Änderungen in den Klebeeigenschaften auf.

Die genannten Eigenschaften des mindestens einseitig selbstklebend ausgerüstetem Trägermaterials legen insbesondere die Verwendung für medizinische Produkte, insbesondere Pflaster, medizinische Fixierungen, Wundabdeckungen, orthopädische oder phlebologische Bandagen und Binden nahe.

Daneben bietet sich die Verwendung eines selbstklebend ausgerüsteten Trägermaterials auch an für technische, reversible Fixierungen, die ohne Beschädigung des Untergrunds (beispielsweise Papier, Kunststoffe, Glas, Textilien, Holz, Metalle oder Mineralien) zu lösen sind.

Im folgenden soll ein erfindungsgemäßes selbstklebend ausgerüstetes Trägermaterial mittels mehrerer Beispiele dargestellt werden, ohne die Erfindung unnötig einschränken zu wollen.

**Beispiel 1:**

Erfindungsgemäß wurde eine unelastische selbstklebende Binde hergestellt, die aufgrund ihrer nachfolgend beschriebenen Eigenschaften zur Anwendung als funktioneller Tapeverband dienen kann, wobei sich die Funktionelle Verbandtechnik an der Anatomie und der Biomechanik orientiert.

Die für diesen Verbandtyp verwendete Binde bestand aus einem unelastischen Baumwollgewebe mit einer Höchstzugkraft von größer 60 N/cm und einer HöchstzugkraftDehnung von kleiner 20 %.

Die Selbstklebemasse wurde im Thermosiebdruck auf den Träger appliziert, wobei es sich bei der Selbstklebemasse um einen Heißschmelzselbsthaftkleber handelte.
Diese Schmelzhaftklebemasse setzte sich wie folgt zusammen:

- ein A-B/A-B-A Blockcopolymer, welches aus harten und weichen Segmenten besteht, mit einem Verhältnis der A-B-A zur A-B von 3:7 und einem Styrolgehalt im Polymer von 30 Mol.-%; der Anteil an der Klebemasse beträgt 64 Gew.-% (Kraton G)
- ein Paraffinkohlenwasserstoffwachs mit einem Anteil an der Klebmasse von 32 Gew.-%
- Kohlenwasserstoffharze mit einem Anteil von 3,5 Gew.-% (Super Resin HC 140)
- ein Alterungsschutzmittel mit einem Anteil von weniger als 0,5 Gew.-% (Irganox)

Die eingesetzten Komponenten wurden in einem Thermomischer bei 175 °C homogenisiert.
Der Erweichungspunkt dieser Klebemasse betrug ca. 95°C (DIN 52011), und die Klebmasse zeigte eine Viskosität von 2400 mPas bei 150 °C (DIN 53018, Brookfield DV II, Sp. 21). Die Glasübergang betrug nach oben genannter Methode -10 °C.
Die direkte Beschichtung erfolgte mit 50 m/min bei einer Temperatur von 120 °C. Das Trägermaterial wurde mit 120 g/m$^2$ punktförmig beschichtet, wobei eine 25 Meshsieb-Schablone mit einer Siebstärke von 300 µm verwendet wurde.
Die nach diesem Verfahren hergestellte Binde zeigte ein reversibles Ablösen von der Haut sowie eine gute Luft- und Wasserdampfdurchlässigkeit. Aufgrund der hohen Scherstabilität des Schmelzhaftklebers wurde eine ausreichend Stabilisierung und eine gute propriorezeptive Wirkung festgestellt. Es wurden keine Hautirritationen und eine vernachlässigbar geringe Epilation nach dem Abnehmen der Binde beobachtet.

**Beispiel 2:**

Durch die offenbarte Erfindung konnte die umweltgerechte Rückgewinnung des oft notwendigen Lösemittels, welche kostenintensive und einen hohen Maschinenaufwand zur Folge hat, eingespart werden.
So wurde eine Binde im Thermosiebdruck mit einem Masseauftrag von 160 g/m$^2$ Klebemasse auf Basis eines Blockcopolymeren beschichtet.
Bei dem Blockcopolymeren handelte es sich um ein Styrol-Ethylen-Butylen-Styrol-Blockcopolymerisat, welches mit Paraffinkohlenwasserstoffenwachs versetzt worden ist. Das Verhältnis war ein Teil Polymer auf ein Teil Paraffinkohlenwasserstoff. Zu dieser Mischung wurden 10 % Polystyrolharz (Amoco 18240) gegeben. Der Kleber enthielt ein Prozent Irganox, ein Alterungsschutzmittel (β-(3,5 Di-t.butyl-4-hydroxyphenyl)-propionsäure-n-octadecylester)), und weitere Kohlenwasserstoffharze und Fettsäureester, welche im Gesamtkleber nur zu geringen Mengen enthalten waren. Der Erweichungspunkt dieser Klebemasse betrug ca. 100 °C (DIN 52011) und die Glasübergangstemperatur, bestimmt nach oben genannter Methode, -6 °C.
Mit einer 14 Mesh Siebschablone mit einem Durchlaß von 51% konnte der hohe Masseauftrag erzielt werden. Durch die Verwendung der großen Beschichtungspunkte konnte eine gute Haftung auf dem Träger und ein sauberes Schneiden erzielt werden. Erfindungsgemäß war die Klebemasse hautverträglich und gut haut- und trägerrückseitenverklebend.
Die so hergestellte Binde war auch in einem mehrlagigen Verband luftdurchlässig (mehr als 15 cm$^3$/(cm$^2$*s) und wasserdampfdurchlässig (mehr als 1500 g/(m$^2$*24h).
Die elastische adhäsive Binde wurde für Kompressions- Stütz und Entlastungsverbände eingesetzt, wobei die hohe Sofort-, Dauerklebkraft und Scherfestigkeit vorteilhaft waren. Die Modellierbarkeit und Anwenderempfindung waren durch die den partiellen Auftrag der Klebemasse verbessert worden.

**Beispiele 3 bis 5:**

In der folgenden Tabelle sind in übersichtlicher Weise weitere Beispiele unterschiedlicher, beschichteter Trägermaterialien angegeben, und zwar jeweils die Meßergebnisse, die mit den wie angegeben beschichteten Trägermaterialien erzielt worden sind.

| Bezeichnung | Beispiel 3 | Beispiel 4 | Beispiel 5 |
|---|---|---|---|
| Trägermaterial | elastisches Baumwollgewebe | starres Baumwollgewebe | starres Zellwollgewebe |
| Siebschablone (Mesh und Siebdicke) | 14 M / 450µm | 14 M / 300 µm | 25 M / 300 µm |

(fortgesetzt)

| Bezeichnung | Beispiel 3 | Beispiel 4 | Beispiel 5 |
|---|---|---|---|
| Trägermaterial | elastisches Baumwollgewebe | starres Baumwollgewebe | starres Zellwollgewebe |
| Glasübergangstemp. in °C (Schmelzhaftselbstklebers) | -16±2°C | -16 ± 2°C | -16 ± 2°C |
| Anfaßklebrigkeit (Schmelzhaftselbstkleber) | tan δ = 0,93 ± 0,03 | tan δ = 0,93 ± 0,03 | tan δ = 0,93 ± 0,03 |
| Scherstabilität (Schmelzhaftselbstkleber) | tan δ = 0,10 ± 0,03 | tan δ = 0,10 ± 0,03 | tan δ = 0,10 ± 0,03 |
| Masseauftrag in $g/m^2$ | 140 | 125 | 82 |
| Klebkraft auf Stahl in N/cm | 8,5 | 10,3 | 6,5 |
| Klebkraft auf der Trägerrückseite in N/cm | 2,3 | 3,5 | 2,5 |
| Luftdurchlässigkeit in $cm^3/(cm^2 \ast s)$ | 96 | 67 | 103 |
| Wasserdampfdurchlässigkeit in $g/(m^2 \ast 24\,h)$ | 2500 | 2100 | 2800 |
| Umspulen auf Haut | nein | nein | nein |
| Hautunverträglichkeiten/ Hautirritationen | keine | keine | keine |

**Patentansprüche**

1. Mindestens einseitig selbstklebend ausgerüstete Trägermaterialien, **dadurch gekennzeichnet, daß** die Selbstklebemasse eine Haftschmelzklebemasse ist, die bei einer Frequenz von 0,1 rad/s eine dynamisch-komplexe Glasübergangstemperatur von weniger als -3 °C, bevorzugt von -6 °C bis -30 °C, ganz besonders bevorzugt von -9 °C bis -25 °C, aufweist.

2. Selbstklebend ausgerüstetes Trägermaterial gemäß Anspruch 1, **dadurch gekennzeichnet, daß** die Klebemasse auf Blockcopolymerbasis aufgebaut ist, insbesondere A-B- oder A-B-A-Blockcopolymere oder deren Mischungen, wobei Phase A vornehmlich Polystyrol oder dessen Derivate und Phase B Ethylen, Propylen, Butylen, Butadien, Isopren oder deren Mischungen, hierbei besonders bevorzugt Ethylen und Butylen oder deren Mischungen, sind.

3. Selbstklebend ausgerüstetes Trägermaterial gemäß Anspruch 1 oder 2, **dadurch gekennzeichnet, daß** der gesamte Styrolanteil im Polymer niedriger als 35 Gew.-%, bevorzugt 5 Gew.-% bis 30 Gew.-%, ist.

4. Selbstklebend ausgerüstetes Trägermaterial gemäß einem der Ansprüche 1 bis 3, **dadurch gekennzeichnet, daß** die Haftschmelzklebemasse besteht aus

   - 10 Gew.-% bis 90 Gew.-%     Blockcopolymere,
   - 5 Gew.-% bis 80 Gew.-%     Klebrigmacher wie Öle, Wachse, Harze und/oder deren Mischungen, bevorzugt Mischungen aus Harzen und Ölen,
   - weniger als 60 Gew.-%     Weichmacher
   - weniger als 15 Gew.-%     Additive
   - weniger als 5 Gew.-%     Stabilisatoren.

5. Selbstklebend ausgerüstetes Trägermaterial gemäß Anspruch 4, **dadurch gekennzeichnet, daß** die eingesetzten Öle, Wachse und Harze Kohlenwasserstofföle, -wachse und -harze sind.

6. Selbstklebend ausgerüstetes Trägermaterial gemäß einem der Ansprüche 1 bis 5, **dadurch gekennzeichnet, daß** der Haftschmelzklebemasse nichtklebende Substanzen, wie mineralische Füllstoffe, zugemischt sind.

7. Selbstklebend ausgerüstetes Trägermaterial gemäß einem der Ansprüche 1 bis 6, **dadurch gekennzeichnet, daß** das Verhältnis des Verlustmoduls (viskoser Anteil) zum Speichermodul (elastischer Anteil) der Haftschmelzklebemasse bei einer Frequenz von 100 rad/s bei 25 °C größer 0,7 ist.

8. Selbstklebend ausgerüstetes Trägermaterial gemäß einem der Ansprüche 1 bis 7, **dadurch gekennzeichnet, daß** das Verhältnis des Verlustmoduls (viskoser Anteil) zum Speichermodul (elastischer Anteil) der Haftschmelzklebemasse bei einer Frequenz von 0,1 rad/s bei 25 °C kleiner 0,40, bevorzugt zwischen 0,35 bis 0,02, ganz besonders bevorzugt zwischen 0,30 und 0,10, ist.

9. Selbstklebend ausgerüstetes Trägermaterial gemäß einem der Ansprüche 1 bis 8, **dadurch gekennzeichnet, daß** die Selbstklebemasse partiell aufgetragen ist.

10. Selbstklebend ausgerüstetes Trägermaterial gemäß einem der Ansprüche 1 bis 9, **dadurch gekennzeichnet, daß** die Haftschmelzklebemasse durch Rasterdruck, Thermosiebdruck oder Tiefdruck aufgebracht ist.

11. Selbstklebend ausgerüstetes Trägermaterial gemäß einem der Ansprüche 1 bis 10, **dadurch gekennzeichnet, daß** die Selbstklebemasse in Form von polygeometrischen Kalotten auf das Trägermaterial gebracht wird.

12. Selbstklebend ausgerüstetes Trägermaterial gemäß einem der Ansprüche 1 bis 11, **dadurch gekennzeichnet, daß** die Selbstklebemasse mit einem Flächengewicht von größer 15 g/m$^2$, bevorzugt zwischen 70 g/m$^2$ und 300 g/m$^2$, ganz besonders bevorzugt zwischen 90 g/m$^2$ und 160 g/m$^2$, auf dem Trägermaterial beschichtet ist.

13. Selbstklebend ausgerüstetes Trägermaterial gemäß einem der Ansprüche 1 bis 12, **dadurch gekennzeichnet, daß** die Selbstklebemasse mit einer Flächendeckung von 20 % bis 95 % aufgebracht ist.

14. Selbstklebend ausgerüstetes Trägermaterial gemäß einem der Ansprüche 1 bis 13, **dadurch gekennzeichnet, daß** das selbstklebend ausgerüstete Trägermaterial eine Klebkraft auf der Trägerrückseite von mindestens 1,5 N/cm, besonders eine Klebkraft zwischen 2,5 N/cm und 5 N/cm, aufweist.

15. Selbstklebend ausgerüstetes Trägermaterial gemäß einem der Ansprüche 1 bis 14, **dadurch gekennzeichnet, daß** das beschichtete Trägermaterial eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), aufweist.

16. Selbstklebend ausgerüstetes Trägermaterial gemäß einem der Ansprüche 1 bis 15, **dadurch gekennzeichnet, daß** das beschichtete Trägermaterial eine Wasserdampfdurchlässigkeit von größer 500 g/(m$^2$*24h), bevorzugt größer 1000 g/(m$^2$*24h), ganz besonders bevorzugt größer 2000 g/(m$^2$*24h), aufweist.

17. Selbstklebend ausgerüstetes Trägermaterial gemäß einem der Ansprüche 1 bis 16, **dadurch gekennzeichnet, daß** das Trägermaterial vorbehandelt ist.

18. Selbstklebend ausgerüstetes Trägermaterial gemäß einem der Ansprüche 1 bis 17, **dadurch gekennzeichnet, daß** das selbstklebend ausgerüstete Trägermaterial nach der Applikation eingedeckt oder mit einer Wundauflage, Polsterung versehen wird.

19. Selbstklebend ausgerüstetes Trägermaterial gemäß einem der Ansprüche 1 bis 18, **dadurch gekennzeichnet, daß** das selbstklebend ausgerüstete Trägermaferial sterilisierbar, bevorzugt γ (gamma)-sterilisierbar, ist.

20. Verwendung eines selbstklebend ausgerüsteten Trägermaterials gemäß einem der Ansprüche 1 bis 19 für medizinische Produkte, insbesondere Pflaster, medizinische Fixierungen, Wundabdeckungen, orthopädische oder phlebologische Bandagen und Binden.

21. Verwendung eines selbstklebend ausgerüsteten Trägermaterials gemäß einem der Ansprüche 1 bis 20 für technische, reversible Fixierungen, die ohne Beschädigung des Untergrunds zu lösen sind.

**Claims**

1. Backing materials which have been given a self-adhesive treatment on at least one side, **characterized in that** the self-adhesive composition is a pressure-sensitive hotmelt adhesive composition which at a frequency of 0.1 rad/s has a dynamic-complex glass transition temperature of less than -3 °C, preferably from -6 °C to -30 °C and, with particular preference, from -9 °C to - 25 °C.

2. Self-adhesively treated backing material according to Claim 1, **characterized in that** the adhesive composition is formulated on the basis of block copolymers, especially A-B or A-B-A block copolymers or mixtures thereof, in which phase A is above all polystyrene or its derivatives and phase B is ethylene, propylene, butylene, butadiene, isoprene or mixtures thereof, with particular preference being given to ethylene and butylene or mixtures thereof.

3. Self-adhesively treated backing material according to Claim 1 or 2, **characterized in that** the overall proportion of styrene in the polymer is less than 35% by weight, preferably from 5% by weight to 30% by weight.

4. Self-adhesively treated backing material according to one of Claims 1 to 3, **characterized in that** the pressure-sensitive hotmelt adhesive composition consists of

| - from 10% by weight to 90% by weight | of block copolymers, |
| - from 5% by weight to 80% by weight | of tackifiers such as oils, waxes, resins and/or mixtures thereof, preferably mixtures of resins and oils, |
| - less than 60% by weight | of plasticizers, |
| - less than 15% by weight | of additives, and |
| - less than 5% by weight | of stabilizers. |

5. Self-adhesively treated backing material according to Claim 4, **characterized in that** the oils, waxes and resins employed are hydrocarbon oils, waxes and resins.

6. Self-adhesively treated backing material according to one of Claims 1 to 5, **characterized in that** nonadhesive substances, such as mineral fillers, are admixed to the pressure-sensitive hotmelt adhesive composition.

7. Self-adhesively treated backing material according to one of Claims 1 to 6, **characterized in that** the ratio of the loss modulus (viscous component) to the storage modulus (elastic component) of the pressure-sensitive hotmelt adhesive composition at a frequency of 100 rad/s at 25 °C is greater than 0.7.

8. Self-adhesively treated backing material according to one of Claims 1 to 7, **characterized in that** the ratio of the loss modulus (viscous component) to the storage modulus (elastic component) of the pressure-sensitive hotmelt adhesive composition at a frequency of 0.1 rad/s at 25 °C is less than 0.40, preferably between 0.35 and 0.02 and, with particular preference, between 0.30 and 0.10.

9. Self-adhesively treated backing material according to one of Claims 1 to 8, **characterized in that** the self-adhesive composition is applied partially.

10. Self-adhesively treated backing material according to one of Claims 1 to 9, **characterized in that** the pressure-sensitive hotmelt adhesive composition is applied by halftone printing, thermal screen printing or intaglio printing.

11. Self-adhesively treated backing material according to one of Claims 1 to 10, **characterized in that** the self-adhesive composition is applied in the form of polygeometric domes to the backing material.

12. Self-adhesively treated backing material according to one of Claims 1 to 11, **characterized in that** the self-adhesive composition is coated on the backing material with a weight per unit area of more than 15 g/m$^2$, preferably between 70 g/m$^2$ and 300 g/m$^2$ and, with particular preference, between 90 g/m$^2$ and 160 g/m$^2$.

13. Self-adhesively treated backing material according to one of Claims 1 to 12, **characterized in that** the self-adhesive composition is applied with an areal coverage of from 20% to 95%.

14. Self-adhesively treated backing material according to one of Claims 1 to 13, **characterized in that** the self-adhesively treated backing material has a bond strength to the reverse of the backing of at least 1.5 N/cm, in particular a bond strength of between 2.5 N/cm and 5 N/cm.

15. Self-adhesively treated backing material according to one of Claims 1 to 14, **characterized in that** the coated backing material has an air permeability of greater than 1 cm$^3$/(cm$^2$*s), preferably greater than 15 cm$^3$/(cm$^2$*s) and, with very particular preference, greater than 70 cm$^3$/(cm$^2$*s).

16. Self-adhesively treated backing material according to one of Claims 1 to 15, **characterized in that** the coated backing material has a water vapour permeability of greater than 500 g/(m$^2$*24h), preferably greater than 1000 g/(m$^2$*24h) and, with particular preference, greater than 2000 g/(m$^2$*24h).

17. Self-adhesively treated backing material according to one of Claims 1 to 16, **characterized in that** the backing material is pretreated.

18. Self-adhesively treated backing material according to one of Claims 1 to 17, **characterized in that** the self-adhesively treated backing material, following application, is covered or is provided with a wound pad or with padding.

19. Self-adhesively treated backing material according to one of Claims 1 to 18, **characterized in that** the self-adhesively treated backing material can be sterilized, preferably by γ (gamma) radiation.

20. Use of a self-adhesively treated backing material according to one of Claims 1 to 19 for medical products, especially plasters, medical fixings, wound coverings, or orthopaedic or phlebological bandages and dressings.

21. Use of a self-adhesively treated backing material according to one of Claims 1 to 20 for reversible technical fixings which can be detached without damage to the substrate.

## Revendications

1. Matériaux support apprêtés au moins d'un côté de manière auto-adhésive, **caractérisés en ce que** la masse auto-adhésive est une masse auto-adhésive fusible qui présente, à une fréquence de 0,1 rad/s une température de transition vitreuse dynamique complexe inférieure à -3°C, de préférence de -6°C à -30°C, de manière particulièrement préférée de -9°C à -25°C.

2. Matériau support apprêté de manière auto-adhésive selon la revendication 1, **caractérisé en ce que** la masse adhésive est réalisée à base de copolymères à blocs, en particulier de copolymères à blocs A-B ou A-B-A ou leurs mélanges, la phase A étant principalement du polystyrène ou ses dérivés et la phase B de l'éthylène, du propylène, du butylène, du butadiène, de l'isoprène ou leurs mélanges, de manière particulièrement préférée l'éthylène et le butylène ou leurs mélanges.

3. Matériau support apprêté de manière auto-adhésive selon les revendications 1 ou 2, **caractérisé en ce que** la proportion totale en styrène dans le polymère est inférieure à 35% en poids, de préférence de 5% en poids à 30% en poids.

4. Matériau support apprêté de manière auto-adhésive selon l'une quelconque des revendications 1 à 3, **caractérisé en ce que** la masse auto-adhésive fusible est constituée de

   - 10% en poids à 90% en poids de copolymères à blocs,
   - 5% en poids à 80% en poids d'adhésif tel que des huiles, des cires, des résines et/ou leurs mélanges, de préférence des mélanges de résines et d'huiles,
   - moins de 60% de plastifiant
   - moins de 15% en poids d'additifs
   - moins de 5% en poids de stabilisateurs.

5. Matériau support apprêté de manière auto-adhésive selon la revendication **4, caractérisé en ce que** les huiles, cires et résines utilisées sont des huiles, cires et résines hydrocarbonées.

6. Matériau support apprêté de manière auto-adhésive selon l'une quelconque des revendications 1 à 5, **caractérisé en ce que** des substances non adhésives, telles que des charges minérales, sont mélangées à la masse auto-adhésive fusible.

**7.** Matériau support apprêté de manière auto-adhésive selon l'une quelconque des revendications 1 à 6, **caractérisé en ce que** le rapport module de perte (proportion visqueuse) à module d'accumulation (proportion élastique) de la masse auto-adhésive fusible, à une fréquence de 100 rads/s à 25°C, est supérieur à 0,7.

**8.** Matériau support apprêté de manière auto-adhésive selon l'une quelconque des revendications 1 à 7, **caractérisé en ce que** le rapport module de perte (proportion visqueuse) à module d'accumulation (proportion élastique) de la masse auto-adhésive fusible, à une fréquence de 0,1 rad/s à 25°C, est inférieur à 0,40, de préférence entre 0,35 et 0,02, de manière particulièrement préférée entre 0,30 et 0,10.

**9.** Matériau support apprêté de manière auto-adhésive selon l'une quelconque des revendications 1 à 8, **caractérisé en ce que** la masse auto-adhésive est appliquée partiellement.

**10.** Matériau support apprêté de manière auto-adhésive selon l'une quelconque des revendications 1 à 9, **caractérisé en ce que** la masse auto-adhésive fusible est appliquée par impression matricielle, par thermosérigraphie ou par héliogravure.

**11.** Matériau support apprêté de manière auto-adhésive selon l'une quelconque des revendications 1 à 10, **caractérisé en ce que** la masse auto-adhésive est appliquée sous forme de calottes poly-géométriques sur le matériau support.

**12.** Matériau support apprêté de manière auto-adhésive selon l'une quelconque des revendications 1 à 11, **caractérisé en ce que** la masse auto-adhésive est revêtue sur le matériau support à une masse au mètre carré supérieure à 15 g/m$^2$, de préférence entre 70 g/m$^2$ et 300 g/m$^2$, de manière particulièrement préférée entre 90 g/m$^2$ et 160 g/m$^2$.

**13.** Matériau support apprêté de manière auto-adhésive selon l'une quelconque des revendications 1 à 12, **caractérisé en ce que** la masse auto-adhésive est appliquée à un recouvrement superficiel de 20% à 95%.

**14.** Matériau support apprêté de manière auto-adhésive selon l'une quelconque des revendications 1 à 13, **caractérisé en ce que** le matériau support apprêté de masse auto-adhésive présente une adhésivité sur le dos du support d'au moins 1,5 N/cm, en particulier une adhésivité entre 2,5N/cm et 5 N/cm.

**15.** Matériau support apprêté de manière auto-adhésive selon l'une quelconque des revendications 1 à 14, **caractérisé en ce que** le matériau support revêtu présente une perméabilité à l'air supérieure à 1 cm$^3$/(cm$^2$*s), de préférence supérieure à 15 cm$^3$/(cm$^2$*s), de manière particulièrement préférée supérieure à 70 cm$^3$/(cm$^2$*s).

**16.** Matériau support apprêté de manière auto-adhésive selon l'une quelconque des revendications 1 à 15, **caractérisé en ce que** le matériau support revêtu présente une perméabilité à la vapeur d'eau supérieure à 500 g/(m$^2$*24 h), de préférence supérieure à 1000 g/(m$^2$*24 h), de manière particulièrement préférée supérieure à 2000 g/(m$^2$*24 h).

**17.** Matériau support apprêté de manière auto-adhésive selon l'une quelconque des revendications 1 à 16, **caractérisé en ce que** le matériau support est prétraité.

**18.** Matériau support apprêté de manière auto-adhésive selon l'une quelconque des revendications 1 à 17, **caractérisé en ce que** le matériau support apprêté de manière auto-adhésive est recouvert ou pourvu d'un tissu chirurgical, d'un rembourrage après l'application.

**19.** Matériau support apprêté de manière auto-adhésive selon l'une quelconque des revendications 1 à 18, **caractérisé en ce que** le matériau support apprêté de manière auto-adhésive peut être stérilisé, de préférence par une stérilisation $\gamma$ (gamma).

**20.** Utilisation d'un matériau support apprêté de manière auto-adhésive selon l'une quelconque des revendications 1 à 19 pour des produits médicaux, en particulier des pansements, des fixations médicales, des recouvrements chirurgicaux, des bandages et des bandes orthopédiques et en cas de phlébites.

**21.** Utilisation d'un matériau support apprêté de manière auto-adhésive selon l'une quelconque des revendications 1 à 20 pour des fixations techniques réversibles, qui peuvent être détachées du substrat sans détérioration.